# EUROPEAN PATENT APPLICATION

(11) **EP 2 832 423 A1**
(43) Date of publication of application: **04.02.2015**
(21) Application number: 13768994.9
(22) Date of filing: 25.03.2013
(51) Int. Cl.: B01D 63/02, A61M 1/18, B01D 63/00

(54) **HOLLOW FIBER MEMBRANE MODULE**

(30) Priority: 28.03.2012 JP 2012073761
(71) Applicant: Toray Industries, Inc., Tokyo, 103-8666 (JP)
(72) Inventor: HASHIMOTO, Kazuyuki, Otsu-shi Shiga 520-8558 (JP); INUZUKA, Hayato, Otsu-shi Shiga 520-8558 (JP); NAKANO, Yoshinori, Okazaki-shi Aichi 444-8522 (JP); NAKAMATSU, Osamu, Okazaki-shi Aichi 444-8522 (JP)
(74) Representative: Prüfer & Partner GbR European Patent Attorneys
(86) International application number: PCT/JP2013/058545
(87) International publication number: WO 2013/146663

(57) **Abstract**

Provided is a hollow fiber membrane module designed to be substantially liquid-tight, to have high strength in welded joints and to be efficiently producible. The hollow fiber membrane module comprises a hollow fiber membrane bundle 4, a main body case 3 housing the hollow fiber membrane bundle 4, membrane fixing layers 5 for fixing the hollow fiber membrane bundle 4 to an inner wall in both ends of the main body case, and headers 8 and 9 attached to both open ends of the case and having an inlet 6 for a liquid to be treated and an outlet 7 for a liquid to be treated, respectively. In the hollow fiber membrane module, each of the headers 8 and 9 and the main body case 3 are ultrasonically welded around the entire circumference in at least two areas, a shear joint is used as a joint design for ultrasonic welding between an outer circumferential surface of the main body case and an inner surface of each header around the entire circumference, and a butt joint is used as a joint design for ultrasonic welding between an inner surface of each header and a corresponding end surface of the main body case around the entire circumference.

## Description

### TECHNICAL FIELD

The present invention relates to a hollow fiber membrane module, and specifically relates to a hollow fiber membrane module suitable for blood purification, a plasmapheresis apparatus, etc.

### BACKGROUND ART

As widely known, a hollow fiber membrane module comprising hollow fiber membranes has been used for blood purification such as hemodialysis. Such a hollow fiber membrane module comprises a cylindrical main body case having an inlet port for a treatment liquid at one end on the outer circumferential surface of the main body case and having an outlet port for a treatment liquid at the other end on the outer circumferential surface, a hollow fiber membrane bundle inserted in the main body case, membrane fixing layers fixing both ends of the hollow fiber membrane bundle onto the inner wall of the main body case, and headers fixed to open ends of the main body case. When the hollow fiber membrane module is used for hemodialysis, a dialysis solution (treatment liquid) is passed through the main body case from the inlet port for a treatment liquid toward the outlet port for a treatment liquid while blood (liquid to be treated) is passed through the hollow fiber membranes from a blood inlet port of the header toward an outlet port. This operation allows successful hemodialysis.

During such a dialysis process, the hollow fiber membrane module is required to be liquid-tightly sealed so as to prevent the liquid to be treated (blood) passing through the hollow fiber membrane module from leaking out through a joint between the header and the main body case. To address the request, joining of the header and the main body case by ultrasonic welding is commonly employed. In a joining method using ultrasonic welding, while a portion to be welded of the header is kept in contact with a portion to be welded of the cylindrical main body case by pressure application to the top face of the header with a tool horn, ultrasonic vibration is applied to transmit the vibration to the portions to be welded, thus allowing both the portions to be welded to generate heat and to be melt joined. The method enables stable sealing of containers which necessitate air-tightness or liquid-tightness, and thus is widely used.

For example, Patent Literature 1 discloses a method for reinforcing a joint between a main body container and a header. The method comprises ultrasonically welding an outer circumference of the main body container to a melting margin on the inside of the header, and then ultrasonically welding the header to the main body container at a side closer to the center of the main body container relative to the melting margin using a tapered horn. The method for reinforcing a joint disclosed in Patent Literature 1 unfortunately necessitates two ultrasonic welding steps, which reduce productivity, and thus is not an efficient method for reinforcing a joint.

The method disclosed in Patent Literature 1 comprises, as shown in Fig. 7, ultrasonically welding a main body container 31 to a header 32 in two areas 33 and 34, but even when the main body container 31 has two ultrasonic welds 33 and 34 around the side surface, blood introduced through a blood inlet port of the header 32 under pressure cannot be prevented from infiltrating into a small gap 36 between the header 32 and a membrane fixing layer 35.

### CITATION LIST

### PATENT LITERATURE

Patent Literature 1: Japanese Patent No. 4842419

### SUMMARY OF INVENTION

### TECHNICAL PROBLEM

In view of the above problems in the conventional art, an object of the present invention is to provide a hollow fiber membrane module designed to be substantially liquid-tight, to have high strength in welded joints and to be efficiently producible.

### SOLUTION TO PROBLEM

To achieve the above object, the inventors of the present invention provide a hollow fiber membrane module comprising a hollow fiber membrane bundle, a main body case housing the hollow fiber membrane bundle, membrane fixing layers for fixing the hollow fiber membrane bundle to an inner wall in both ends of the main body case, and headers attached to both open ends of the case, one header having an inlet for a liquid to be treated, another header having an outlet for a liquid to be treated, each header and the main body case being ultrasonically welded around the entire circumference in at least two areas to form a liquid-tight structure,
the hollow fiber membrane module being characterized by using a shear joint as a joint design for ultrasonic welding between an outer circumferential surface of the main body case and an inner surface of each header around the entire circumference, and using a butt joint as a joint design for ultrasonic welding between an inner surface of each header and a corresponding end surface of the main body case around the entire circumference.

Here, the ultrasonic welding obviously provides a joint without any defects such as bubbles and cracks, and makes the inner circumferential surface of the header in contact with the membrane fixing layer around the entire circumference.

The shear joint preferably has a cross-sectional area of 0.3 to 0.9 mm² and more preferably 0.4 to 0.7 mm². The cross-sectional area is calculated by multiplying a width of the joint by a height of the joint. A shear joint having a cross-sectional area of less than 0.3 mm² is unlikely to provide sufficient joint strength. A shear joint having a cross-sectional area of more than 0.9 mm² requires higher energy for melting and thus is harder to melt. The shear joint preferably has a width of 0.25 mm or more. When having a width of less than 0.25 mm, the shear joint of the header elastically deforms in the outer circumferential direction. Such a header hardly overlaps the main body case and thus substantial welding may be impossible.

The distance in the horizontal direction between an innermost circumference of the butt joint and an inner circumferential surface of the header is preferably 0.5 to 2.0 mm and more preferably 1.0 to 2.0 mm. As the distance in the horizontal direction becomes shorter, the butt joint improves the liquid-tightness, but the distance is required to be at least 0.5 mm due to technical limitations. When the distance is more than 2.0 mm, the butt joint is unlikely to improve the liquid-tightness.

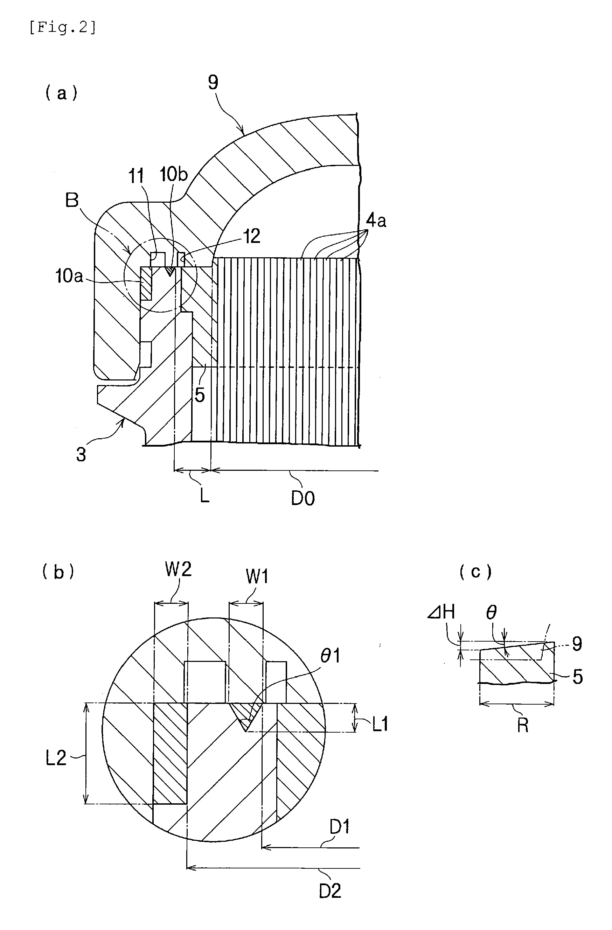

The area of a triangle in the butt joint is preferably 0.05 to 0.35 mm² and more preferably 0.1 to 0.2 mm². The area is calculated by multiplying a width of a director by one-half a height of the director. The triangle area means the cross-sectional area of a joint in the director. When the area is less than 0.05 mm², the director cannot help the header and the main body case melt sufficiently and will be merely pressed against the end surface of the case, thus failing to provide sufficient joint strength. When the area is more than 0.35 mm², the director requires higher energy for melting and thus is harder to melt. The director preferably has a tip angle (θ1 in Fig. 2b) of 40 to 70° because such a tip angle allows a stress to be concentrated to the tip so that the director easily melts.

The hollow fiber membrane module preferably further comprises a resin receiver at an inner circumferential side of the butt joint and another resin receiver at an outer circumferential side of the butt joint. The resin receivers receive a melted resin and thereby contribute to good results in ultrasonic welding.

In a method for producing a hollow fiber membrane module including a hollow fiber membrane bundle, a main body case housing the hollow fiber membrane bundle, membrane fixing layers for fixing the hollow fiber membrane bundle to an inner wall in both ends of the main body case, and headers attached to both open ends of the case, one header having an inlet for a liquid to be treated, another header having an outlet for a liquid to be treated, each header and the main body case are ultrasonically welded around the entire circumference in at least two areas at once, a shear joint is used to ultrasonically weld an outer circumferential surface of the main body case and an inner surface of each header around the entire circumference, and a butt joint is used to ultrasonically weld an inner surface of each header and a corresponding end surface of the main body case around the entire circumference.

In a hollow fiber membrane module produced by inserting a bundle of a plurality of hollow fiber membranes made of a polymer for a hollow fiber membrane into a main body case, attaching caps for casting a membrane fixing layer to both ends of the main body case, injecting a polymer for a membrane fixing layer into the ends of the main body case to form membrane fixing layers for fixation of the hollow fiber membrane bundle to the main body case, partially cutting the solidified membrane fixing layers to make both end surfaces of the hollow fiber membrane bundle open toward the outside, and ultrasonically welding headers and the main body case having partially cut membrane fixing layers therein, the ultrasonically welding being performed between each header and the main body case around the entire circumference in at least two areas at once, an uncut end surface of each membrane fixing layer is in contact with an inner surface of the corresponding header.

### ADVANTAGEOUS EFFECTS OF INVENTION

The present invention can provide a hollow fiber membrane module designed to be substantially liquid-tight, to have high strength in welded joints and to be efficiently producible.

### BRIEF DESCRIPTION OF DRAWINGS

Fig. 1 is a sectional side view showing an embodiment of a hollow fiber membrane module of the present invention.
Fig. 2a is an enlarged partial view of Fig. 1 showing a principal part of the present invention; Fig. 2b is an enlarged view of the part encircled in Fig. 2a; and Fig. 2c is a view illustrating a reverse taper of a membrane fixing layer to come in contact with the inner surface of a header.
Fig. 3 is a schematic sectional view showing an example of a shear joint.
Fig. 4 is a schematic sectional view showing an example of a butt joint.
Fig. 5 is a schematic side view showing an exemplary ultrasonic welder.
Fig. 6 is a sectional side view showing a main body case having an end surface installed with a cap.
Fig. 7 is a sectional side view showing an example of a hollow fiber membrane module for illustrating the method for reinforcing a joint disclosed in Patent Literature 1.

### DESCRIPTION OF EMBODIMENTS

A hollow fiber membrane module according to the present invention can be applied to medical modules such as hemodialyzers typified by a hollow fiber membrane-based artificial kidney, plasma separators, plasma component separators, blood filters, plasma component adsorbers, artificial lungs, and endotoxin removing filters; adsorption modules packed with adsorbents; modules for filter apparatuses; and the like.

An embodiment of the hollow fiber membrane module of the present invention will be described with reference to drawings.

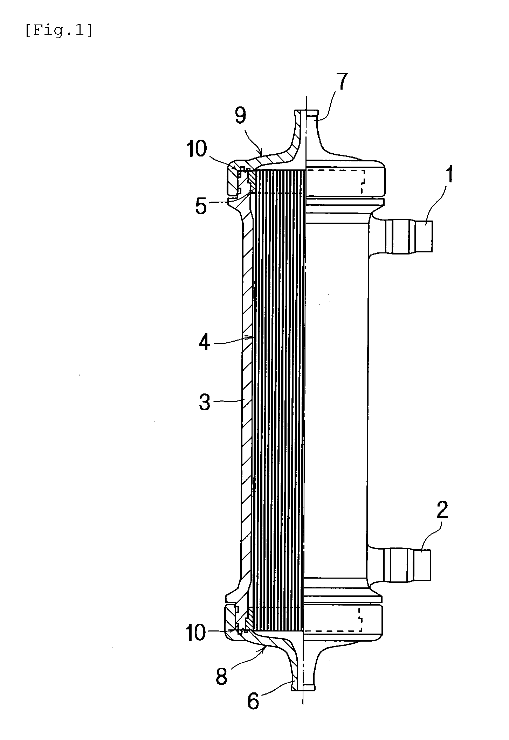

Fig. 1 shows an embodiment of the hollow fiber membrane module of the present invention used for hemodialyzers, and preferred aspects and examples are not intended to be limited to the embodiment. In Fig. 1, a hollow fiber membrane module comprises a cylindrical main body case 3 that has an inlet port 1 for a treatment liquid in the vicinity of one end in the longitudinal direction and an outlet port 2 for a treatment liquid in the vicinity of the other end, a hollow fiber membrane bundle 4 that is inserted in the main body case 3 and is prepared by aligning a large number of hollow fiber membranes in one direction, and membrane fixing layers 5 that fix the hollow fiber membrane bundle 4 to an inner wall in both ends of the main body case 3. To the both ends of the main body case 3, a header 8 as a cover having an inlet port 6 for blood as a liquid to be treated and a header 9 as a cover having an outlet port 7 for blood as a liquid to be treated are fixed by welding through ultrasonic welds 10.

Examples of the materials for the main body case and the header include, but are not limited to, plastics such as polypropylene, polyethylene, polyester, polytetrafluoroethylene, polycarbonate, and acrylonitrile butadiene styrene (ABS). Thermosetting resins have a three-dimensional molecular structure, and once molded, they do not melt even when heat is applied. Thermoplastic resins are chain polymers, and even after molded, they melt when heat is applied. In the present invention, the thermoplastic resins are thus preferably used. The thermoplastic resins are classified into amorphous resins and semi-crystalline resins. The amorphous resins are composed of a polymer chain, but the semi-crystalline resins partially have regular molecular arrangements in a polymer chain, and thus the amorphous resins are easier to use for ultrasonic welding than the semi-crystalline resins (for example, polyamide, polyethylene, polypropylene, and polyacetal).

Examples of the material for the hollow fiber membrane bundle include cellulose, cellulose derivatives, polymethyl methacrylate (PMMA), polypropylene, and polysulfone. However, when a hydrophobic polymer such as polysulfone is used alone to produce a hollow fiber membrane used for dialysis or other purposes, the pore size is difficult to control. In addition, the hydrophobicity causes blood components such as blood platelets to adhere to the hollow fiber membrane, and this is disadvantageous in blood compatibility. The problems can be solved by a combination use of a hydrophobic polymer with a hydrophilic polymer. In a specific example, a hydrophilic polymer is previously added as a pore forming agent to a stock solution for producing membranes, some of the hydrophilic polymer is removed to form pores, and then the remaining hydrophilic polymer is used to render a hydrophilic polymer surface. The thus-prepared product can also be used as a hollow fiber membrane.

Examples of the hydrophilic polymer include polyethylene glycol, polyvinyl alcohol, carboxymethylcellulose, and polyvinylpyrrolidone, and these polymers may be used alone or as a mixture. Polyvinylpyrrolidone is preferred because it is relatively easily available in an industrial scale, has been clinically used, and has high blood compatibility. Examples of the material for the membrane fixing layer include polymer materials such as polyurethane, silicone, or epoxy polymers. Preferably used are two-pack type curable polymer adhesives containing any of these polymer materials. The membrane fixing layer can be produced by centrifugal molding (potting) or the like.

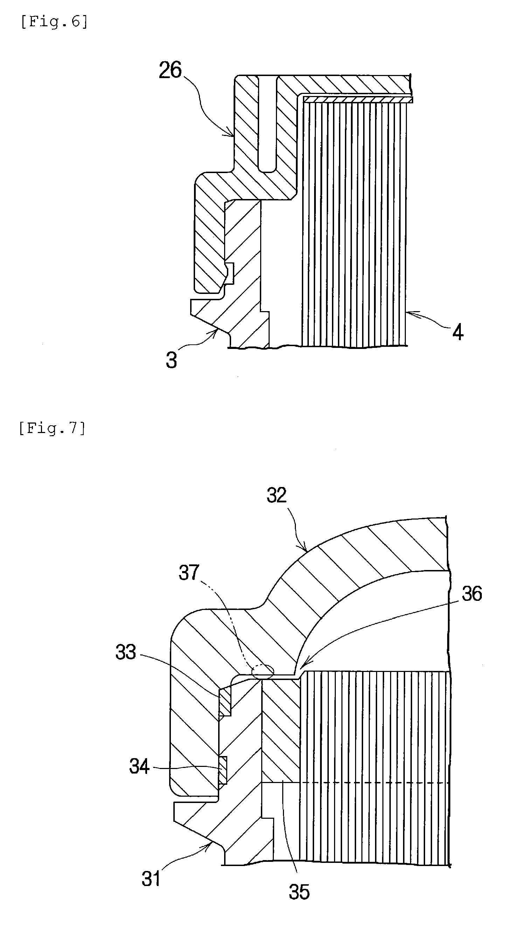

A method for producing a hollow fiber membrane module from the materials listed above will be described with reference to Fig. 1. A plurality of hollow fiber membranes made of a polymer for a hollow fiber membrane selected from the materials listed above are bundled and inserted into a main body case 3 molded by known injection molding or another method. To both ends of the main body case 3, caps 26 for casting a membrane fixing layer 5 (for example, see Fig. 6) are attached, and then a polymer for a membrane fixing layer selected from the materials listed above is injected into the ends of the main body case 3 to form the membrane fixing layers 5 for fixation of the hollow fiber membrane bundle 4 to the main body case 3. The membrane fixing layers 5, after solidified, are partially cut so as to make both end surfaces of the hollow fiber membrane bundle 4 open toward the outside. To the both ends of the main body case 3 having partially cut membrane fixing layers therein, headers 8 and 9 molded by known injection molding or another method are liquid-tightly attached by ultrasonic welding to give a hollow fiber membrane module.

The present invention has an important feature in which each header and the main body case are ultrasonically welded around the entire circumference in at least two areas to form a liquid-tight structure, and the important feature of the present invention will be described with reference to Fig. 2a. In the following description, the ultrasonic welding between the main body case 3 and the header 9 will be described. The ultrasonic welding between the main body case 3 and the header 8 can also be performed in the same manner, and thus the overlapping description will be omitted.

As shown in Fig. 2a, the main body case 3 and the header 9 form a liquid-tight structure owing to an ultrasonic weld 10a composed of a shear joint around the entire circumference and an ultrasonic weld 10b composed of a butt joint around the entire circumference. The ultrasonic weld 10a and the ultrasonic weld 10b can be simultaneously formed by ultrasonic welding, and thus the present invention can efficiently produce a hollow fiber membrane module. In drawings, 11 and 12 are resin receivers, which can receive a resin melted by ultrasonic welding and thereby the ultrasonic welding smoothly advances. Appropriate volumes of the resin receivers 11 and 12 vary depending on shapes of the butt joint and the shear joint and thus are not particularly specified, but are appropriately determined so that the resin receivers can store a resin melted from the both joints.

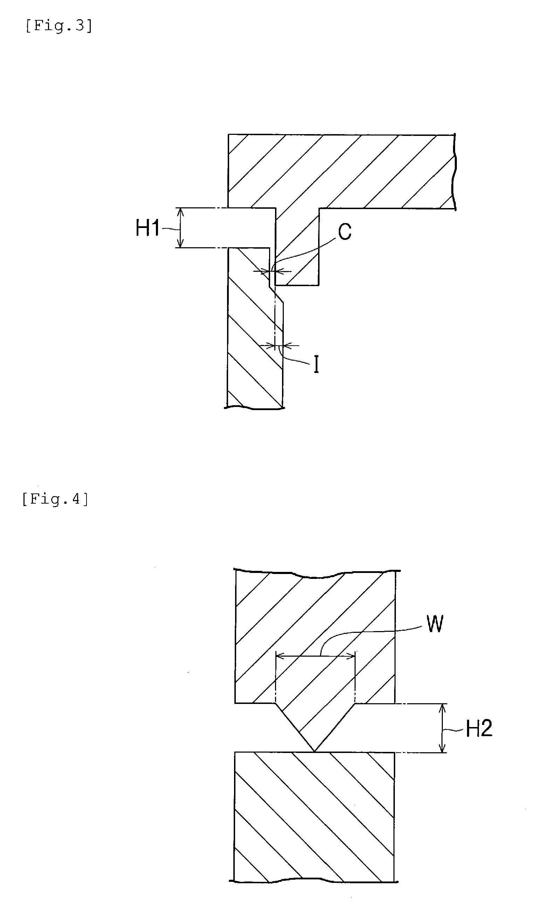

To form a liquid-tight structure by ultrasonic welding, a joint design is very important. Examples of the joint design includes a scarf joint, which intends to give complete surface contact on inclined planes; a beet joint, which allows uniform heat generation at a joint by surface contact in a vibration direction; a shear joint, which is an intermediate type between the scarf joint and the beet joint as shown in Fig. 3 and allows a vibration direction and a contact surface to approach in the same direction with respect to the longitudinal vibration transmitted by a horn, thus enabling prevention of bubble generation on a weld surface and providing excellent liquid-tightness and air-tightness; and a butt joint, which is one of the joint designs using an energy director as shown in Fig. 4 and allows efficient welding by concentrating energy to a triangular protrusion called a director to raise the temperature to a resin melting temperature in a very short period of time.

In Fig. 3, H1 is a welding margin, C is a clearance between an upper work and a lower work, and I is an interference size. In Fig. 4, W is a width of the director, and H2 is a height of the director. The butt joint advantageously has a simple shape and requires a relatively small space for a joint, but may fail to provide strong ultrasonic wave energy in longitudinal vibration welding. In contrast, the shear joint does not have such a disadvantage, and when used as the joint design for a semi-crystalline resin that requires strong ultrasonic wave energy, the shear joint can provide high weld strength and liquid-tightness. In the present invention, by using both the shear joint and the butt joint having such respective advantages, high joint strength and excellent liquid-tightness can be achieved.

In Fig. 2b, the shear joint preferably has a width (W2) of 0.25 mm or more. The cross-sectional area calculated by multiplying the width (W2) by the height (L2) of the shear joint is preferably 0.3 to 0.9 mm² and more preferably 0.4 to 0.9 mm² for the reasons described above.

In Fig. 2a, the distance (L) in the horizontal direction between the innermost circumference of the ultrasonic weld 10b composed of the butt joint and the inner circumferential surface of the header is preferably 0.5 to 2.0 mm for the reasons described above.

In Fig. 4, the area of a triangle (the cross-sectional area of the joint part of the butt joint) is preferably 0.05 to 0.35 mm² for the reasons described above. The area is calculated by multiplying the width (W) by one-half the height (H2) of the director.

The hollow fiber membrane module of the present invention is produced by inserting a bundle of a plurality of hollow fiber membranes made of a polymer for a hollow fiber membrane into a main body case, attaching caps 26 for casting a membrane fixing layer to both ends of the main body case, injecting a polymer for a membrane fixing layer into the ends of the main body case to form membrane fixing layers for fixation of the hollow fiber membrane bundle to the main body case, partially cutting the solidified membrane fixing layers to make both end surfaces of the hollow fiber membrane bundle open toward the outside, and ultrasonically welding headers and the main body case having partially cut membrane fixing layers therein, the ultrasonically welding being performed between each header and the main body case around the entire circumference in at least two areas at once. In the production process, as shown in Fig. 2a, the end surface of the membrane fixing layer 5 through which a plurality of the hollow fiber membranes 4a are inserted is cut so as to make both end surfaces of the hollow fiber membrane bundle 4 open toward the outside, and the cut end surface of the membrane fixing layer 5 is slightly rougher than the uncut end surface of the membrane fixing layer 5. As shown in Fig. 2a, when the inner surface of the header 9 is in contact with the uncut, smooth end surface of the membrane fixing layer 5, the liquid-tightness is further improved. As an indicator for smoothness of the membrane fixing layer 5, the center-line average surface roughness Ra is preferably 50 µm or less and more preferably 20 µm or less. When a membrane fixing layer 5 has a wavy end surface, blood may infiltrate into a gap between such a surface and the inner circumferential surface of the header 9. On this account, the membrane fixing layer 5 to come in contact with the inner circumferential surface of the header 9 preferably has a height difference of 100 µm or less around the entire circumference. When the membrane fixing layer 5, in a region to come in contact with the inner surface of the header 9, has a reverse taper that gradually heightens from the outer circumferential side toward the center in the radial direction, the repulsion between the header 9 and the membrane fixing layer 5 is so high as to more probably prevent blood infiltration. The reverse taper preferably has an angle (θ (ΔH/R) in Fig. 2c) of 1 to 10° and more preferably 2 to 6°.

As described above, in the hollow fiber membrane module of the present invention, the ultrasonic welding performed between the header and the main body case around the entire circumference in at least two areas by using a shear joint known for its excellent versatility and a butt joint known for its efficiency can improve the liquid-tightness and increase the strength of the welded joints. As shown in Fig. 7, in a conventional method for joining a header and a main body case, in order to prevent blood from infiltrating into a gap 36 between a header 32 and a membrane fixing layer 35, an elastic ring 37 is placed as a seal material on the header 32. The hollow fiber membrane module of the present invention has excellent liquid-tightness and thus does not need the elastic ring 37.

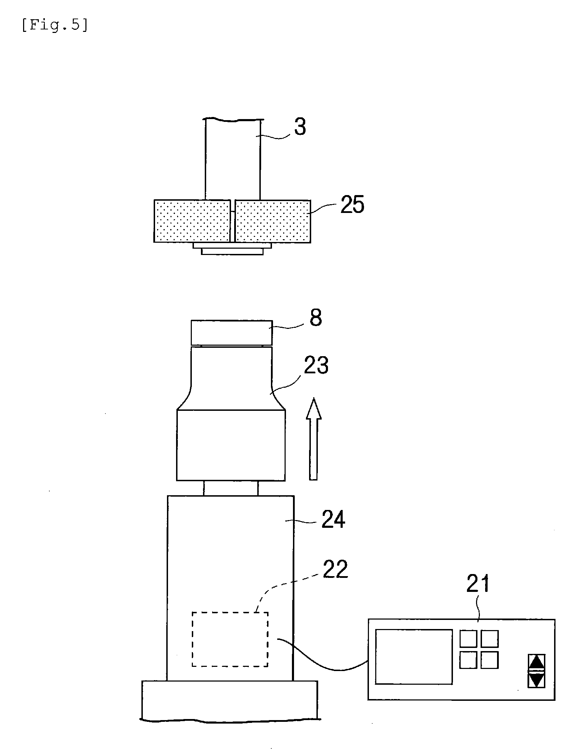

In the present invention, any ultrasonic welder can be used, and preferred is a welder generating ultrasonic waves having a frequency of 15 kHz to 60 kHz. A welder generating ultrasonic waves having a frequency of about 15 kHz to 40 kHz more readily transmits vibration to portions to be welded and thus can provide good results in transmission welding. An exemplary welder is shown in Fig. 5. In Fig. 5, an oscillator 21 generates a signal, which vibrates a vibrator 22. The vibration is amplified into a predetermined amplitude by a horn 23. An actuator 24 pushes the horn 23 against a header 8, and under the pressure on the top face of the header, the horn 23 transmits the ultrasonic vibration to the ultrasonic weld 10 in Fig. 1.

The portion to be welded of the header strikes the portion to be welded of the main body case 3 by pressure application with the horn 23. Then, application of ultrasonic vibration under the pressure rapidly generates heat and starts melting at the interface between the portions to be welded. The melted resin flows to a peripheral space. Further application of pressure and ultrasonic vibration advances the melting of the portions to be welded. When the melted amount of the portions to be welded reaches an intended amount, the ultrasonic vibration is terminated. The portions to be welded are then allowed to cool, resulting in the fixation of the header 8 to the main body case 3.

The welding pressure is not particularly specified because an appropriate welding pressure varies with materials of the header and the main body case and shapes of the portions to be welded. The welding pressure is typically about 0.2 to 0.6 MPa for circumferential welding of a cylindrical container having a diameter of 60 mm. In the case of a header made of polypropylene or polyethylene, an excessively large welding pressure may cause the header to deform and may generate heat in a portion except the portions to be welded, and thus a lower welding pressure than that for other materials is used for good welding results.

In Fig. 5, a hollow fiber membrane module is held by a holder 25. By holding a hollow fiber membrane module by the holder 25 as shown in Fig. 5, vibration can be uniformly transmitted and thus stable welding results can be obtained. The welder may be disposed in any direction. For example, the horn is disposed below the main body case, or the horn is disposed above the main body case. In each case, the header and the main body case are preferably aligned on the same axis to stabilize welding conditions.

### EXAMPLES

Examples of the present invention will be described hereinafter, but the present invention is not intended to be limited by the following examples, and various modifications and changes may be made without departing from the technical scope of the present invention.

### Example 1

Into a polypropylene case (main body case, see sign 3 in Fig. 1) having a full-length of 285 mm, a trunk inner diameter of 34.8 mm, an end inner diameter of 44.2 mm, and an end outer diameter of 48.2 mm, a hollow fiber membrane bundle prepared by bundling 9,200 polysulfone hollow fiber membranes having an inner diameter of 200 µm and a membrane thickness of 40 µm was inserted so that each end of the bundle would protrude 7 mm or more from the corresponding end of the main body case. To the end surface of each end of the bundle, a carbon dioxide gas laser was applied at an output power of 80 W in a predetermined pattern to seal the hollow portions on each end surface of the hollow fiber membrane. Next, on each end of the main body case, a cap (see sign 26 in Fig. 6) was placed. By using the centrifugal force generated by rotation of the main body case around an axis passing through the center of the full-length of the main body case at a radius which is one-half the full-length of the main body case, a urethane resin injected through an inlet port for a treatment liquid (see sign 1 in Fig. 1) and an outlet port for a treatment liquid (see sign 2 in Fig. 1) was cured to form membrane fixing layers in the both ends of the main body case, and thus the hollow fiber membrane bundle was fixed to the main body case. The membrane fixing layer had a diameter of 44.2 mm at the case end. The diameter was straightly reduced to 41 mm from the case end to 0.1 mm outside, and the membrane fixing layer extended from the position to up to 7.4 mm outside. However, in the vicinity of the inner circumferential surface of the main body case end, the membrane fixing layer had a protrusion having a length of less than 0.5 mm from the case end to the outside. Such a membrane fixing layer was cut at a position 0.5 mm outside from the end of the main body case in a direction orthogonal to the axis direction of the main body case. Thus, the end surface of the membrane fixing layer was formed, and the hollow fiber membrane was made to open. The main body case was then ultrasonically welded to polypropylene headers (see signs 8 and 9 in Fig. 1) under conditions of a frequency of 20 kHz, a welding pressure of 0.35 MPa, a welding time of 0.7 second, and a holding time of 1.0 second. Here, the ultrasonic welding was performed while the inner circumferential surface of the header was pressed against the uncut surface around the outer side of the cut surface in the end surface of the membrane fixing layer, that is, against the membrane fixing layer surface formed along the cap inner surface. This is because, when the inner circumferential surface of the header is pressed against the cut surface, there partially exists a gap between the mating surfaces due to a wavy surface resulting from the cutting. When the inner circumferential surface of the header is pressed against the membrane fixing layer surface formed along the cap inner surface, such a gap is reduced and thus ultrasonic welding between the surfaces in closer contact with each other is possible.

In the example, as shown in Fig. 6, the cap 26 used has a shape that allows the formation of a membrane fixing layer in which a portion of the end surface in the vicinity of the outer circumference protrudes less than 0.5 mm from the end of the main body case, and thus when the membrane fixing layer is cut at a position 0.5 mm outside from the end of the main body case 3, the membrane fixing layer in the vicinity of the outer circumference remains uncut and the membrane fixing layer surface formed along the cap inner surface is left as it is.

An intermediate product obtained by welding was disassembled. The header (see sign 9 in Fig. 2a) had an inner circumferential surface diameter D0 of 41.5 mm; the first joint (see sign 10b in Fig. 2a) had a leading end diameter D1 of 44.6 mm, a weld depth L1 of 0.5 mm, and a weld width W1 of 0.5 mm; the second joint (see sign 10a in Fig. 2a) had an inner diameter D2 of 47.4 mm, a weld depth L2 of 1.2 mm, and a weld width W2 of 0.4 mm; the first resin receiver (see sign 12 in Fig. 2a) had a width of 0.5 mm and a height of 1 mm; and the second resin receiver (see sign 11 in Fig. 2a) had a width of 0.9 mm and a height of 1 mm (see Fig. 2b). The second joint had a joint width of 0.4 mm; the second joint had a cross-sectional area of 0.48 mm²; the horizontal distance between an innermost circumference of the first joint and an inner circumferential surface of the header was 1.55 mm; and the first joint had a cross-sectional area of 0.125 mm². Finally, to an inlet port for a liquid to be treated (see sign 6 in Fig. 1), an outlet port for a liquid to be treated (see sign 7 in Fig. 1), the inlet port for a treatment liquid (see sign 1 in Fig. 1) and the outlet port for a treatment liquid (see sign 2 in Fig. 1), plugs were attached. The whole was packed and sterilized by γ-ray, and a blood treatment apparatus having an effective membrane area of 1.5 m² was completed.

A flow channel for a treatment liquid of the blood treatment apparatus produced as above was filled with a dialysis solution, and a flow channel for a liquid to be treated was filled with a mixed liquid containing India ink and physiological saline at a ratio of 1:10. To the inlet port for a treatment liquid (see sign 1 in Fig. 1), the outlet port for a treatment liquid (see sign 2 in Fig. 1), and the outlet port for a liquid to be treated (see sign 7 in Fig. 1), plugs were liquid-tightly attached. To the inlet port for a liquid to be treated (see sign 6 in Fig. 1), a manual pressure pump was connected through a tube. With the pressure pump, the blood treatment apparatus was pressurized at an internal pressure of 10 kPa for 10 seconds, and the operation of increasing the internal pressure by 10 kPa was repeated until the infiltration of the liquid into the interface between the headers (see signs 8 and 9 in Fig. 1) and the membrane fixing layers (see sign 5 in Fig. 1) was observed. The internal pressure at which the infiltration was observed was considered as an infiltration-resisting pressure. The evaluation was carried out on five blood treatment apparatuses produced as above. The average infiltration-resisting pressure was 508 kPa, and the minimum infiltration-resisting pressure was 220 kPa.

### Example 2

Into a polypropylene main body case having a full-length of 285 mm, a trunk inner diameter of 45.4 mm, an end inner diameter of 54.8 mm, and an end outer diameter of 58.8 mm, a hollow fiber membrane bundle prepared by bundling 16,000 polysulfone hollow fiber membranes having an inner diameter of 200 µm and a membrane thickness of 40 µm was inserted so that each end of the bundle would protrude 7 mm or more from the corresponding end of the main body case. To the end surface of each end of the bundle, a carbon dioxide gas laser was applied at an output power of 80 W in a predetermined pattern to seal the hollow portions on each end surface of the hollow fiber membrane. Next, on each end of the main body case (see sign 3 in Fig. 1), a cap (see Fig. 6) was placed. By using the centrifugal force generated by rotation of the main body case around an axis passing through the center of the full-length of the main body case at a radius which is one-half the full-length of the main body case, a urethane resin injected through an inlet port for a treatment liquid (see sign 1 in Fig. 1) and an outlet port for a treatment liquid (see sign 2 in Fig. 1) was cured to form membrane fixing layers in the both ends of the main body case, and thus the hollow fiber membrane bundle was fixed to the main body case. The membrane fixing layer had a diameter of 54.8 mm at the case end. The diameter of 54.8 mm was maintained from the case end to 1 mm outside. From the position to 0.1 mm outside, the diameter was straightly reduced to 51.6 mm, and the membrane fixing layer extended from the position to up to 6.4 mm outside. However, in the vicinity of the inner circumferential surface of the main body case end, the membrane fixing layer had a protrusion having a length of less than 1.5 mm to the outside. Such a membrane fixing layer was cut at a position 1.5 mm outside from the end of the main body case in a direction orthogonal to the axis direction of the main body case. Thus, the end surface of the membrane fixing layer was formed, and the hollow fiber membrane was made to open. The main body case was then ultrasonically welded to polypropylene headers (see signs 8 and 9 in Fig. 1) under conditions of a frequency of 20 kHz, a welding pressure of 0.4 MPa, a welding time of 0.7 second, and a holding time of 1.0 second. Also in Example 2, the ultrasonic welding was performed while the inner circumferential surface of the header was pressed against the uncut surface around the outer side of the cut surface in the end surface of the membrane fixing layer, in other words, against the membrane fixing layer surface formed along the cap inner surface, in the same manner as in Example 1.

An intermediate product obtained by welding was disassembled. The header (see sign 9 in Fig. 2a) had an inner circumferential surface diameter D0 of 52.1 mm; the first joint (see sign 10b in Fig. 2a) had a leading end diameter D1 of 55.0 mm, a weld depth L1 of 0.5 mm, and a weld width W1 of 0.5 mm; the second joint (see sign 10a in Fig. 2a) had an inner diameter D2 of 58 mm, a weld depth L2 of 1.2 mm, and a weld width W2 of 0.4 mm; the first resin receiver (see sign 12 in Fig. 2a) had a width of 0.4 mm and a height of 0.7 mm; and the second resin receiver (see sign 11 in Fig. 2a) had a width of 1 mm and a height of 0.7 mm (see Fig. 2b). The second joint had a joint width of 0.4 mm; the second joint had a cross-sectional area of 0.48 mm²; the horizontal distance between an innermost circumference of the first joint and an inner circumferential surface of the header was 1.45 mm; and the first joint had a cross-sectional area of 0.125 mm². Finally, to an inlet port for a liquid to be treated (see sign 6 in Fig. 1), an outlet port for a liquid to be treated (see sign 7 in Fig. 1), the inlet port for a treatment liquid (see sign 1 in Fig. 1) and the outlet port for a treatment liquid (see sign 2 in Fig. 1), plugs were attached. The whole was packed and sterilized by γ-ray, and a blood treatment apparatus having an effective membrane area of 2.6 m² was completed.

Five blood treatment apparatuses were subjected to the same pressure-resisting test as in Example 1. The average infiltration-resisting pressure was 262 kPa, and the minimum infiltration-resistant pressure was 130 kPa.

### Comparative Example 1

A blood treatment apparatus was completed in the same manner as in Example 1 by using a header having the same shape as in Example 1 except that the header had no first joint (see sign 10b in Fig. 2a). The second joint had a joint width of 0.4 mm; the cross-sectional area calculated by multiplying a width by a height of the second joint was 0.48 mm²; and the horizontal distance between an innermost circumference of the second joint and an inner circumferential surface of the header was 2.95 mm. Five blood treatment apparatuses were subjected to the same pressure-resistant test as in Example 1. The average infiltration-resisting pressure was 30 kPa, and the minimum infiltration-resisting pressure was 0 kPa.

### Comparative Example 2

A blood treatment apparatus was completed in the same manner as in Example 1 by using a header having the same shape as in Example 1 except that the first joint (see sign 10b in Fig. 2a) had a leading end diameter D1 of 45 mm, a weld depth L1 of 0.7 mm, and a weld width W1 of 1.1 mm, the header had no second joint (see sign 10a in Fig. 2a), the first resin receiver (see sign 12 in Fig. 2a) had a width of 0.6 mm and a height of 0.8 mm, and the second resin receiver (see sign 11 in Fig. 2a) had a width of 0.6 mm and a height of 0.8 mm. The first joint had a cross-sectional area of 0.493 mm²; and the horizontal distance between an innermost circumference of the first joint and an inner circumferential surface of the header was 1.75 mm. Five blood treatment apparatuses were subjected to the same pressure-resistant test as in Example 1. The average infiltration-resisting pressure was 69 kPa, and the minimum infiltration-resisting pressure was 0 kPa.

### Comparative Example 3

A blood treatment apparatus was completed in the same manner as in Example 1 by using a header having the same shape as in Example 1 except that the header had neither a first resin receiver (see sign 12 in Fig. 2a) nor a second resin receiver (see sign 11 in Fig. 2a). The second joint had a joint width of 0.4 mm; the second joint had a cross-sectional area of 0.4 mm²; the horizontal distance between an innermost circumference of the first joint and an inner circumferential surface of the header was 1.55 mm; and the first joint had a cross-sectional area of 0.075 mm². Five blood treatment apparatuses were subjected to the same pressure-resistant test as in Example 1. The infiltration was observed at 0 kPa in each of five pressure-resistant tests. When the blood treatment apparatus was cut in the longitudinal direction, the melted resin had permeated between the header and the end surface of the main body case, and a 0.1 to 0.2-mm gap was observed between the inner circumferential surface of the header and the membrane fixing layer.

### Comparative Example 4

A blood treatment apparatus was completed in the same manner as in Example 1 except that a cap having a shape different from that in Example 1 was used to form a membrane fixing layer. The formed membrane fixing layer had a diameter of 44.2 mm at the main body case end, extended from the case end to up to 7.5 mm outside, and had a protrusion having a length of 0.5 mm or more from the case end to the outside in the vicinity of the inner circumferential surface of the case end. This membrane fixing layer was cut at a position 0.5 mm outside from the case end in a direction orthogonal to the axis direction of the main body case, and ultrasonic welding of headers to the main body case was then performed while the inner circumferential surface of the header was pressed against the cut end surface of the resulting membrane fixing layer. In other words, unlike Examples 1 and 2 and Comparative Examples 1 to 3, in Comparative Example 4 alone, the inner circumferential surface of the header was pressed against the cut surface of the membrane fixing layer during the ultrasonic welding. The second joint had a joint width of 0.4 mm; the second joint had a cross-sectional area of 0.48 mm²; the horizontal distance between an innermost circumference of the first joint and an inner circumferential surface of the header was 1.55 mm; and the first joint had a cross-sectional area of 0.125 mm². Five blood treatment apparatuses were subjected to the same pressure-resistant test as in Example 1. The infiltration was observed at 0 kPa in each of five pressure-resistant tests. When the infiltrated portion was cut in the longitudinal direction, a 0.1 to 0.3-mm gap was observed between the inner circumferential surface of the header and the membrane fixing layer.

### Comparative Example 5

A blood treatment apparatus was completed in the same manner as in Example 1 by using the same header as in Example 2 except that the first joint had a leading end diameter D1 of 55.2 mm, a weld depth L1 of 0.3 mm, and a weld width W1 of 0.3 mm. The second joint had a joint width of 0.4 mm; the second joint had a cross-sectional area of 0.48 mm²; the horizontal distance between an innermost circumference of the first joint and an inner circumferential surface of the header was 1.55 mm; and the first joint had a cross-sectional area of 0.045 mm². Five blood treatment apparatuses were subjected to the same pressure-resistant test as in Example 1. The average infiltration-resisting pressure was 55 kPa, and the minimum infiltration-resisting pressure was 0 kPa. When the infiltrated portion was cut in the longitudinal direction, the first joint had been merely deformed by compression, and the header and the case were not welded practically.

### Comparative Example 6

A blood treatment apparatus was completed in the same manner as in Example 2 except that the polypropylene main body case was heated in an oven at a temperature of 50°C for 30 minutes to reduce the end inner diameter to 54.4 mm and the end outer diameter to 58.4 mm. The second joint had a joint width of 0.2 mm; the second joint had a cross-sectional area of 0.24 mm²; the horizontal distance between an innermost circumference of the first joint and an inner circumferential surface of the header was 1.55 mm; and the first joint had a cross-sectional area of 0.125 mm². However, the second joint partially contained bubbles, and proper welding was not achieved in the apparatus. Five blood treatment apparatuses were subjected to the same pressure-resistant test as in Example 1. The average infiltration-resisting pressure was 48 kPa, and the minimum infiltration-resisting pressure was 10 kPa.

### REFERENCE SIGNS LIST

- 1: Inlet port for a treatment liquid
- 2: Outlet port for a treatment liquid
- 3: Main body case
- 4: Hollow fiber membrane bundle
- 4a: Hollow fiber membrane
- 5: Membrane fixing layer
- 6: Inlet port for a liquid to be treated
- 7: Outlet port for a liquid to be treated
- 8: Header
- 9: Header
- 10: Ultrasonic weld
- 10a: Ultrasonic weld composed of a shear joint
- 10b: Ultrasonic weld composed of a butt joint
- 11: Resin receiver
- 12: Resin receiver
- 21: Oscillator
- 22: Vibrator
- 23: Horn
- 24: Actuator
- 25: Holder
- 26: Cap
- 31: Main body container
- 32: Header
- 33: Ultrasonic weld
- 34: Ultrasonic weld
- 35: Membrane fixing layer
- 36: Gap
- 37: Elastic ring

## Claims

1. A hollow fiber membrane module comprising:
a hollow fiber membrane bundle;
a main body case housing the hollow fiber membrane bundle;
membrane fixing layers for fixing the hollow fiber membrane bundle to an inner wall in both ends of the main body case; and
headers attached to both open ends of the case, one header having an inlet for a liquid to be treated, another header having an outlet for a liquid to be treated,
each header and the main body case being ultrasonically welded around the entire circumference in at least two areas to form a liquid-tight structure,
the hollow fiber membrane module being **characterized by**:
using a shear joint as a joint design for ultrasonic welding between an outer circumferential surface of the main body case and an inner surface of each header around the entire circumference, and
using a butt joint as a joint design for ultrasonic welding between an inner surface of each header and a corresponding end surface of the main body case around the entire circumference.

2. The hollow fiber membrane module according to claim 1, wherein the shear joint has a cross-sectional area of 0.3 to 0.9 mm², the cross-sectional area being calculated by multiplying a width of the joint by a height of the joint.

3. The hollow fiber membrane module according to claim 1, wherein the shear joint has a cross-sectional area of 0.4 to 0.9 mm², the cross-sectional area being calculated by multiplying a width of the joint by a height of the joint.

4. The hollow fiber membrane module according to any one of claims 1 to 3, wherein the distance in the horizontal direction between an innermost circumference of the butt joint and an inner circumferential surface of the header is 0.5 to 2.0 mm.

5. The hollow fiber membrane module according to any one of claims 1 to 4, wherein the area of a triangle in the butt joint is 0.05 to 0.35 mm², the area being calculated by multiplying a width of a director by one-half a height of the director.

6. The hollow fiber membrane module according to any one of claims 1 to 5, further comprising a resin receiver at an inner circumferential side of the butt joint and another resin receiver at an outer circumferential side of the butt joint.

7. A method for producing a hollow fiber membrane module including a hollow fiber membrane bundle, a main body case housing the hollow fiber membrane bundle, membrane fixing layers for fixing the hollow fiber membrane bundle to an inner wall in both ends of the main body case, and headers attached to both open ends of the case, one header having an inlet for a liquid to be treated, another header having an outlet for a liquid to be treated,
the method comprising:
ultrasonically welding each header and the main body case around the entire circumference in at least two areas at once,
using a shear joint to ultrasonically weld an outer circumferential surface of the main body case and an inner surface of each header around the entire circumference, and
using a butt joint to ultrasonically weld an inner surface of each header and a corresponding end surface of the main body case around the entire circumference.

8. A hollow fiber membrane module produced by inserting a bundle of a plurality of hollow fiber membranes made of a polymer for a hollow fiber membrane into a main body case, attaching caps for casting a membrane fixing layer to both ends of the main body case, injecting a polymer for a membrane fixing layer into the ends of the main body case to form membrane fixing layers for fixation of the hollow fiber membrane bundle to the main body case, partially cutting the solidified membrane fixing layers to make both end surfaces of the hollow fiber membrane bundle open toward the outside, and ultrasonically welding headers and the main body case having partially cut membrane fixing layers therein, the ultrasonically welding being performed between each header and the main body case around the entire circumference in at least two areas at once,
the hollow fiber membrane module being **characterized in that** an uncut end surface of each membrane fixing layer is in contact with an inner surface of the corresponding header.
